# EUROPEAN PATENT APPLICATION

(11) **EP 1 176 212 A1**
(43) Date of publication of application: **30.01.2002**
(21) Application number: 00402112.7
(22) Date of filing: 24.07.2000
(51) Int. Cl.: C12Q 1/68

(54) **Method for haplotyping by mass spectrometry**

(71) Applicant: Centre National de Genotype, 91000 Evry (FR)
(72) Inventor: Gut, Ivo Glynne, 75014 Paris (FR); Lechner, Doris, 75012 Paris (FR)
(74) Representative: Texier, Christian

(57) **Abstract**

The invention relates to a method for performing haplotyping of multiple single nucleotide polymorphisms (SNPs) that uses allele specific PCR and mass spectrometry analysis;

## Description

The invention relates to a method for performing haplotyping of multiple single nucleotide polymorphisms (SNPs) that uses allele specific PCR and mass spectrometry analysis.

The complete sequence of the human genome will be achieved and completely published in the next few months. This project will reveal the complete sequence of the 3 billion bases and the relative positions of all estimated (from 30.000 to over 100.000) genes in this genome. Having this sequence opens numerous possibilities for the elucidation of gene function and interaction of different genes.

It also allows the implementation of pharmacogenetics and pharmacogenomics. Pharmacogenetics and pharmacogenomics aim at a targeted use of medication dependent on the genotype of an individual and so the dramatic improvement of the efficiency of drugs. A necessary intermediate step to this is the determination of variability of different individuals on a genome basis. This is accomplished by determining different markers and then using these for genotyping (characterization of the presence of a marker in an individual) and haplotyping (linkage between different markers in close proximity).

Currently two kinds of markers are used for genotyping: microsatellites and single nucleotide polymorphisms (SNPs).

Microsatellites are highly polymorphic markers where different alleles are made up of different numbers of repetitive sequence elements between conserved flanking regions. On average a microsatellite is found every 100.000 bases. A complete map of microsatellite markers covering the human genome was presented by the CEPH (Dib et al., *Nature* 1996 Mar 14;380(6570):152-4). Microsatellites are commonly genotyped by sizing PCR products generated over the repeat region on gels. The most widely used systems are based on the use of fluorescently labeled DNA and their detection in fluorescence sequencers.

Fewer SNPs are currently in the public domain. A SNP map with 300.000 SNPs is being established by the SNP consortium (*Science,* 1999, 284, 406-407).

For genotyping SNPs, there are a few methods available for the person skilled in the art, all of them with advantages and disadvantages.

Some of these methods rely on gel-based detection, like the oligonucleotide ligase assay (OLA), and for this reason only allows medium throughput applications.

Others rely on pure hybridization which is not as discriminating and is difficult to tune to get the high stringency required (oligonucleotide arrays, DNA chips). Although DNA chips are well suited for simultaneous genotyping of a large number of genotypes in a very limited region of the genome and on an overseeable number of individuals, the main problem seen with the use of these objects is the difficulty to optimize the hybridization conditions (in particular for the stringency).

Approaches using primer extension and detection by fluorescence have been shown. Their advantage is facile emission detection in an ELISA type reader. The limitation of these methods is the limited number of fluorescent dyes available, which in return limits the number of sample that can be simultaneously analyzed.

Several methods of SNP genotyping use mass spectrometric detection, as mass spectrometry allows for very high throughput and at the same time gives added information on the base that is present through the mass of the obtained product. In applications where an allele specific product is measured this is direct information and therefore very strong.

Several methods using mass spectrometry have been proposed for SNP genotyping (WO98/23774, US5,843,669, these documents being incorporated herein by reference).

Matrix-assisted laser desorption/ionization time-of-flight mass spectrometry (MALDI) allows the mass spectrometric analysis of biomolecules (Karas and Hillenkamp *Anal*. *Chem.* 60, 2299-2301 (1988)). Indeed, MALDI has been applied to the analysis of DNA in variations that range from the analysis of PCR products to approaches using allele specific termination to single nucleotide primer extension reactions, sequencing and hybridization (US5,885,775, WO96/29431, US5,691,141, WO97/37041, WO94/16101, WO96/27681, GB2339279, all incorporated herein by reference).

Major drawbacks of these approaches are that they heavily rely on stringent purification procedures prior to MALDI analysis that do not lend themselves to easy automation and make up a major part of the cost. Spin column purification and/or magnetic bead technology and reversed-phase purification are frequently applied.

Indeed, the analysis of nucleic acids by MALDI is strongly dependent on the charge state and a 100-fold increase in analysis sensitivity can be achieved when the DNA is conditioned to carry one positive charge. Such modified DNA products are also significantly less susceptible to adduct formation and so do not require purification procedures (WO 96/27681, GB 2339279, Gut and Beck (1995) *Nucleic Acids Research*, **23***,* 1367-1373, Gut *et al*. (1997) *Rapid Commun*. *Mass Spectrom.,* **11**, 43-50, all these documents being incorporated herein by reference).

An assay developed from this for the generation of allele specific products for SNPs has been termed the "GOOD Assay" for SNP analysis (Sauer *et al., Nucleic Acids Research,* 2000, 28, E 13, which is incorporated herein by reference).

Nevertheless, the genotyping information on its own does not allow full assessment of the translation of a DNA sequence into a protein or the regulation of the transcription. In particular, when the two alleles of a given genes carry different SNPs, it is very important to have information about the combination of different SNPs in relation to each other (haplotyping), and about which of the alleles are on the same DNA strand.

There are a few methods for haplotyping. They rely on the generation of allele specific products by allele specific PCR, using a primer whose 3' end base specifically matches one allele to be amplified. Yet, they are limited in their capacity to query multiple positions simultaneously. The presence or absence of a PCR product is used for the identification of a haplotype.

To increase the specificity of allele specific PCR, two major approaches are taken. One is the addition of GC rich tails to the 5'end of the primers for the PCR and doing the PCR with a high annealing temperature (Liu *et al*. (1997), *Genome Res* 7(4): 389-98, which is incorporated herein by reference). For initial cycles of the PCR high stringency is so obtained. In later cycles the GC tail provides a preference for the amplification templates. However, this does not give sufficient stringency in all cases.

Another way to increase the stringency of the allele specific PCR is the introduction of further mismatches (Newton *et al*. (1989), *Nucleic Acids Res* 17(7): 2503-16, which is incorporated herein by reference). This method on its own also may give limited stringency.

It may therefore prove interesting to combine these two methods for increasing the specificity and stringency in the allele specific PCR reaction.

Nevertheless, the allele specific PCR (with or without improvements) has the disadvantage that it only can query two polymorphisms in relation to each other.

Clark *et al*. (1998, *Am*. *J. Hum. Genet*., 63, 595-612) describe a method for the analysis of nucleotide-sequence variation in the Human Lipoprotein Lipase that uses sequencing of the genes and of the allele specific PCR products as the method of analysis for genotyping. The authors develop on the weaknesses of this method of haplotyping, in particular as a lot of effort is required.

It is the aim of the invention to provide a simple and high throughput method for haplotyping, that allows determination of linkage of multiple SNPs in a fast, cost-efficient and reliable way. The method of the invention allows the simultaneous analysis of multiple polymorphous sites, after performing only one allele specific PCR reaction.

Indeed, it is often required to determine the alleles of more than two single nucleotide polymorphisms by genotyping. If it turns out that the individual genotype is heterozygous for more than one of the SNPs, it is interesting to determine which of the alleles are on the same DNA strand.

The invention uses allele specific PCR for amplification of only one allele from the genomic DNA. The allele specific primer is designed to match one allele of a heterozygous SNP. The product of amplification is then genotyped which reveals allows to deduct what the other alleles are on this product and allows the determination of the haplotype, as the previously heterozygous SNPs now appear homozygous.

The association of the polymorphism underlying the allele specific PCR with the determined alleles of the alleles of the other polymorphisms give the haplotype.

The invention is therefore drawn to a method for the determination of the haplotype of an individual, comprising the steps of:
a) genotyping of more than two single nucleotide polymorphisms (SNPs) by mass spectrometry;
b) allele specific PCR with one primer being specific for one allele of a heterozygous polymorphism, if more then one polymorphisms is heterozygous;
c) genotyping on the allele specific PCR product by mass spectrometry.

The method of the invention could also be used to identify nearly identical sequences in order to find out whether a sequence is duplicated or heterozygous. The variations can be used to generate "allele specific" products if other polymorphisms that were heterozygous in the initial genotyping remain heterozygous it is clear that a sequence is duplicated. If the second round genotyping of this systems results in all homozygous SNPs it is probable that the sequence that is being studied is not duplicated.

The use of mass spectrometry allows to perform the analysis of a large number of samples, and obtain the corresponding data in a multiplex reaction. Therefore, the method of the invention that is characterized by a combination between allele specific PCR and the use of mass spectrometry for genotyping and data analysis can be used at high throughput. It can also be automated and will allow an easy and quick determination of the SNP profile of the patients. It will therefore allow the full implementation of pharmaco genetics and pharmacogenomic and improved use of the data obtained from the genome sequencing project.

The genotyping of the SNPs in steps a) and c) is performed by mass spectrometry after generation of allele specific products, which can conventionally be obtained by primer extension, oligonucleotide ligation, cleavase reaction.

One of the advantages of the method according to the invention is the possibility to perform the analysis of multiple SNPs in a DNA sample at the same time in a multiplexed reaction, as known by the person skilled in the art, by choosing the appropriate conditions.

In order to perform the allele specific PCR reaction of step b), one would use a primer that matches one allele of an heterozygous SNP, and preferably a primer that specifically hybridizes with the heterozygous SNP that is located at the most 5' or the most 3' location of all tested SNPs. The other primer would hybridize both alleles and be located such as to obtain the amplification of the region containing all heterozygous SNPs.

In a preferred implementation of the invention, allele specificity for the PCR amplification is achieved by the 3'end base of the primer. This base is, chosen to match one allele and not the other. Further specificity can be achieved by using a primer that has between 10 and 25 bases complementary to the sequence of the genomic DNA, most preferably between 15 and 20 or 22, the specificity being obtained by the 3' end base, as described.

One could also include an unspecific CG rich tail on the 5' end of the primer, and/or further mismatches before the 3' end allele specific base, as described. More preferably, the primer has one mismatch more than 3 bases away from the 3' end.

The annealing temperature is chosen critical (higher than the calculated melting temperature). In the first rounds of the PCR only the fully complementary sequence can anneal. Once some rounds of PCR have been achieved, the higher annealing temperature due to the GC rich tail ensures majority amplification of a single allele.

Mass spectrometry is used for this procedure as is well suited to the analysis of up to several tens of polymorphisms and is very facile in operation. Full automation of the sample preparation is therefore possible by this method. Depending on the sample preparation procedure used for mass spectrometric genotyping, this technology is very effective.

In a preferred implementation of this invention, the method performed for one or both the genotyping steps ( a) and c) ) uses primers that are chimeric in nature, and the procedure followed is the GOOD assay described by Sauer *et al.* (*op*. *cit*., which is incorporated herein by reference).

In a preferred implementation of the invention wherein matrix-assisted laser desorption / ionization time-of-flight mass spectrometry (MALDI) is used for the analysis of the genotypes. In another embodiment, electrospray ionization mass spectrometry is used for the detection.

In a preferred implementation of the invention the reagents for the initial genotyping are the same as the ones used for the genotyping after allele specific PCR.

This invention provides a facile procedure for determining haplotypes that is cost efficient, highly reliable and that can easily be automated, and so lends itself to high-throughput.

This streamlined procedure makes use of the potential of a highly parallel preparation of products for genotyping, their conditioning so that they require no purification and the potential of mass spectrometers to distinguish large numbers of products simultaneously in one spectrum and being able to record a single spectrum in a few seconds. This invention outlines possibilities to dramatically solve the problems for haplotyping a large number of SNPs as currently encountered in the art and makes streamlined and efficient SNP genotyping possible.

The invention further relates to a kit for the implementation of haplotyping by the method of the invention, that comprises primers for PCR that generate allele specific products. The kit of the invention may also include the reagents to perform steps a) and c) of the procedure (generation of samples to be analyzed by mass spectrometry for genotyping), and the instructions as how to perform the method of the invention.

### DESCRIPTION OF THE FIGURES

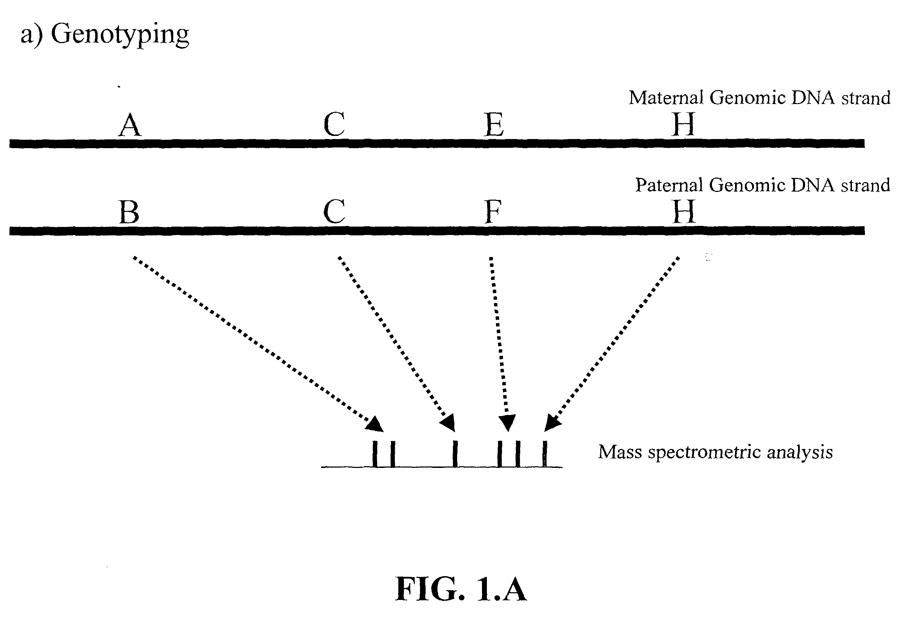

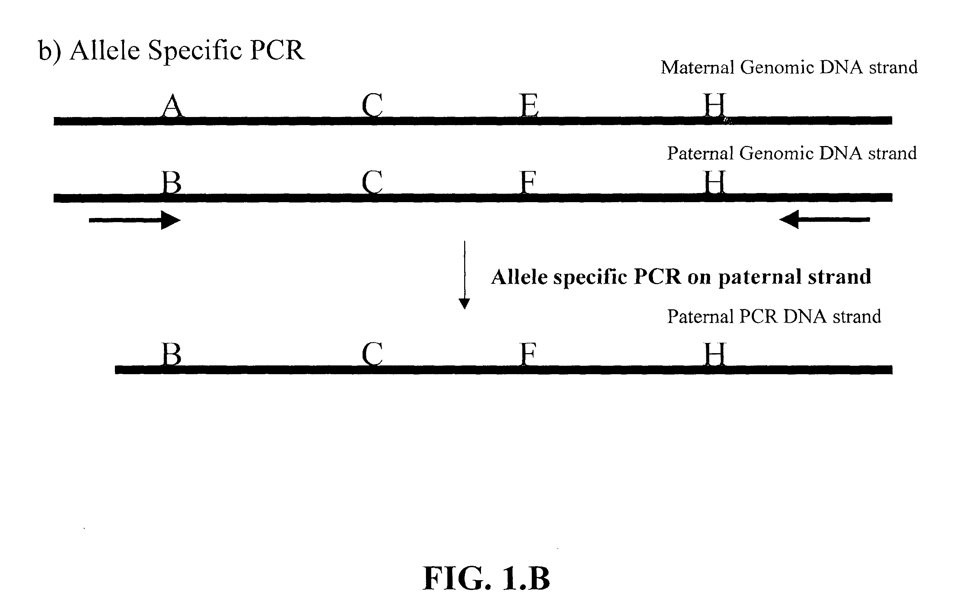

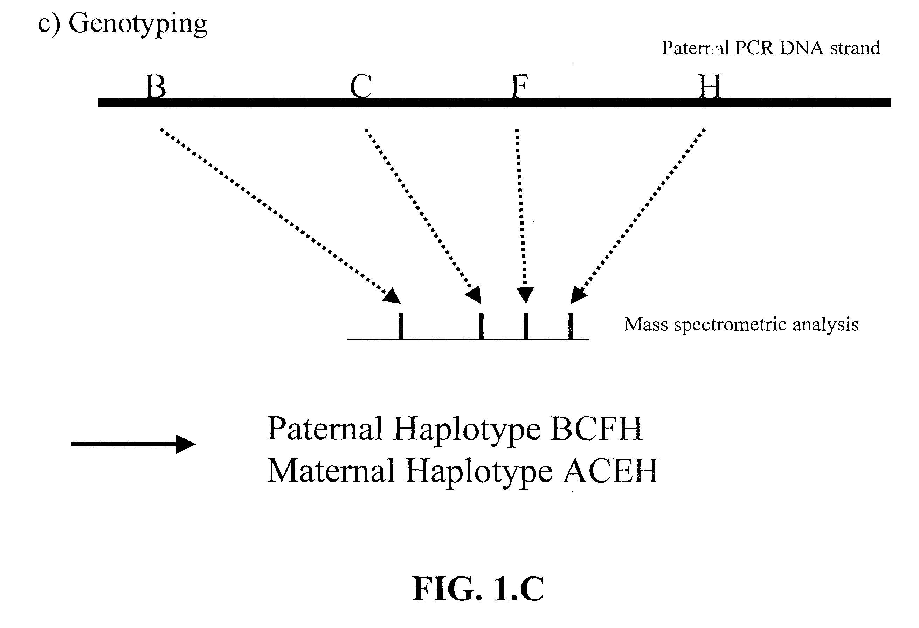

Figure 1 describes the principle of the method of the invention, applied for 4 SNPs, two of them being heterozygous. The first genotyping step (1.A) leads to the generation of 6 products as determined by mass spectrometry. Allele specific PCR (1.B) leads to the amplification of the paternal strand. Genotyping of this product (1.C) allows the identification of the SNPs that are present on this allele.

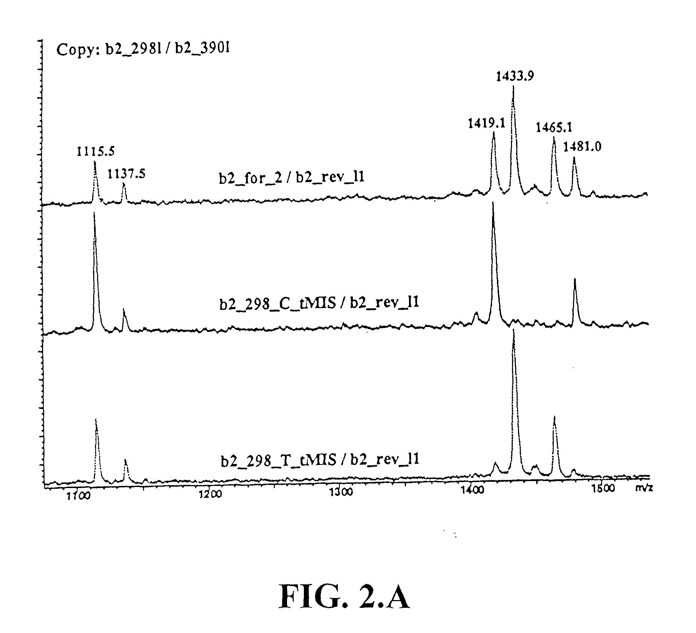

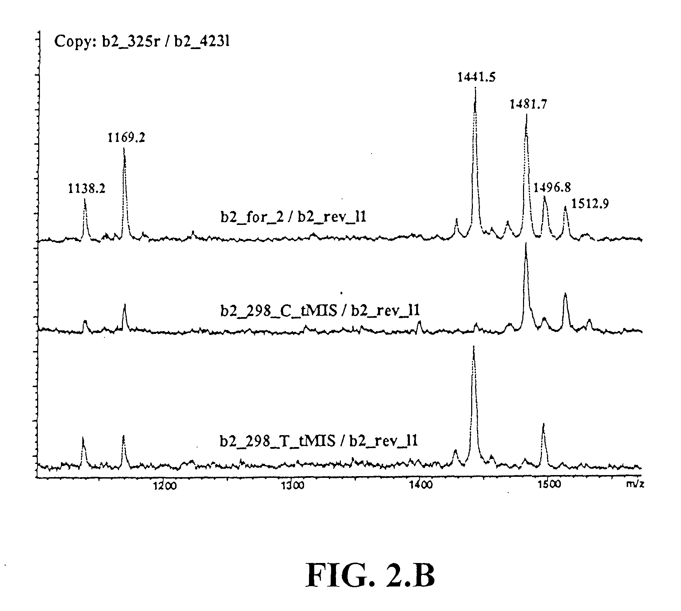

Figure 2 shows typical mass spectrometer spectra obtained after genotping and haplotyping for SNPs 298 and 390 (figure 2.A) and 325 and 423 (figure 2.B) of the beta-2 adrenergic receptor gene. The top spectra shows the genotyping result, while the middle and bottom spectra show the results obtained respectively for haplotype 1 and 2, after allele specific PCR.

### EXAMPLE

The example illustrate the method of the invention and can easily be generalized by the person skilled in the art, for other genes.

The example shown here is the haplotyping of 4 published SNPs in the β2-adrenergic receptor gene. The SNP are T/C at 298, C/T at 325, G/A at 390 and G/C at 423. For each SNP, genotyping by the GOOD assay was established on an amplified (with primers SEQ ID N° 1 and SEQ ID N° 2) fragment of the genomic DNA. The genotyping was performed by following the method of Sauer *et al*., (*Nucleic Acids Research,* 2000, 28, E 13, which is incorporated herein by reference), with primers SEQ ID N° 5 to SEQ ID N° 8, for the primer extension. The analysis is done in positive ion mode on a MALDI mass spectrometer.

In a first experiment the genotype for all four SNPs is determined. The genotype for SNP 298 and SNP 390 is shown on the panel of figure 2.A, while the genotype for SNP 325 and SNP 423 is shown on the panel of figure 2.B. The m/z observed for the products are in the range of 1400 to 1500 Da (figure 2, top spectra).

The data shows that the individual, whose DNA is tested, is heterozygous for the four SNPs.

In order to determine the haplotype, allele specific PCR reactions are carried on, using the allele specific primers SEQ ID N° 3 or SEQ ID N° 4, in combination with the primer SEQ ID N° 2.

Primers SEQ ID N° 3 and SEQ ID N° 4 are specific of one allele of SNP 298 (which is the most 5' of the heterozygous SNPs), and further carry a GC-rich tail, and a mismatch located 5 bases from the 3' end of the primers.

The allele specific PCR could also have been carried out with primer SEQ ID N° 1 and primers that are allele specific for SNP 423 (the most 3' of the heterozygous SNPs).

Genotyping is performed on the allele specific products, (Figure 2, middle spectra for haplotype 1, bottom spectra for haplotype 2), by using the same method as before.

It is clear that the two haplotype obtained add up to the genotype of the individual, and the data allows the determination of the complete haploytpe of the tested individual.

| | SNP 298, -47 (C/T) | SNP 325, -20 (C/T) | SNP 390, 46 (A/G) | SNP 423, 79 (C/G) |
|---|---|---|---|---|
| DNA Sample | MALDI | MALDI | MALDI | MALDI |
| Genotype | TC | TC | GA | GC |
| Haplotype 1 | C | C | G | G |
| Haplotype 2 | T | T | A | C |

The PCR reaction is performed with classical conditions, with 0.5 µl genomic DNA (50 ng/µl), 0.5 µl of each primers SEQ ID N° 1 and SEQ ID N° 2 (7.5 pmol/µl)and the cycling conditions :
1.95 °C 2 min
2. 95 °C 20 sec
3. 68 °C 30 sec
4. 72 °C 30 sec
repeat steps 2 to 4, for 35 times.

Primer extension reactions are classically performed using 1 µl of the copy primer (SEQ ID N° 5 to SEQ ID N° 8) (25 pmol/µl), with the cycling conditions:
1.95 °C 3 min
2.95 °C 10 sec
3.58 °C 30 sec
4.72 °C 15 sec
repeat steps 2 to 4, for 35 times

Phosphodiesterase digest is performed by adding 1 µl acetic acid (0.5 M) and 3 µl PDE are added and incubation at 37 °C for 80 min.

Alkylation is performed by addition of 45 µl acetonitrile, 15 µl triethylamine/CO2 buffer (2 M, pH 8.0) and 14 µl MeI, and incubation at 40°C for 25 min. A sample of 20 µl is taken and mixed with 45 µl of 40% acetonitrile.

MALDI analysis is performed with α-cyano cinnamic acid methyl ester in acetone spotted onto the target, and 0.5 µl of the sample spotted onto the matrix.

Analysis is done in positive ion mode on a MALDI mass spectrometer.

Allele specific PCR is performed using either primer SEQ ID N° 3 or SEQ ID N° 4 and SEQ ID N° 2, following the same classical conditions as previously described.

## Claims

1. A method for the determination of the haplotype of an individual, comprising the steps of:
d) genotyping of more than two single nucleotide polymorphisms (SNPs) by mass spectrometry;
e) allele specific PCR with one primer being specific for one allele of a heterozygous polymorphism, if more then one polymorphisms is heterozygous;
f) genotyping on the allele specific PCR product by mass spectrometry.

2. The method of claim 1, wherein the genotyping of the SNPs in step a), step c) or both steps is performed after generation of allele specific products, said generation of allele specific products being done by primer extension, oligonucleotide ligation, or a cleavase reaction.

3. The method of claim 1 or 2, wherein the genotyping for multiple SNPs in step a), step c) or both steps is performed in one reaction in a multiplexed procedure.

4. The method of any of claims 1 to 3, wherein the allele specific PCR reaction in step b) is achieved by choosing one primer that matches one allele of a heterozygous SNP.

5. The method of claim 4, wherein said primer is chosen as to specifically hybridize with the heterozygous SNP located at the most 5' or the most 3' location of all tested SNPs.

6. The method of any of claims 1 to 5, wherein at least one primer used for the allele specific PCR is fully complementary to the sequence of one allele.

7. The method of any of claims 1 to 6, wherein the 3'end base of the allele specific primer specifically matches one allele of the heterozygous SNP.

8. The method of any of claims 6 to 7 in which said allele specific primer has 10 to 25 bases that are complementary to the sequence of said one allele of the genomic DNA.

9. The method of any of claims 4, 5, 7 and 8, wherein said allele specific primer has a 5'tail that is rich in G and C.

10. The method of any of claims 4, 5, 7 to 9 wherein said allele specific primer has one mismatch in the complementary sequence more then 3 bases away from the 3'end.

11. The method of any of claims 1 to 10 wherein matrix-assisted laser desorption / ionization time-of-flight mass spectrometry is used for either of or both steps a) and c) as defined in claim 1.

12. The method of any of claims 1 to 11 wherein the primers used for generation of the products detected in the genotyping in steps a) and/or c) are chimeric in nature.

13. The method of claim 12, wherein the GOOD assay is applied for either of or both the genotyping steps.

14. The method of any of claims 1 to 13 wherein electrospray ionization mass spectrometry is used for either or both steps a) and c) of claim 1.

15. Kit for the implementation of haplotyping by the method according any of claims 1 to 14 comprising primers for PCR that generate allele specific products.


SEQUENCE LISTING

<110> CENTRE NATIONAL DE GENOTYPAGE

<120> METHOD FOR HAPLOTYPING BY MASS SPECTROMETRY

<130> D19042

<160> 8

<170> PatentIn Ver. 2.1

<210> 1
<211> 19
<212> DNA
<213> Homo sapiens

<220>
<223> Primer b2_for_2 for amplification of the region of
      the beta-2 adrenergic receptor gene.

<400> 1
ctcgcgggcc cgcagagcc                                                19

<210> 2
<211> 24
<212> DNA
<213> Homo sapiens

<220>
<223> Primer b2_rev_11 for amplification of the region
      of the beta-2 adrenergic receptor gene.

<400> 2
gttggtgacc gtctgcagac gctc                                          24

<210> 3
<211> 26
<212> DNA
<213> Artificial Sequence

<220>
<223> Description of Artificial Sequence: Allele
      specific primer b2_298_C_tMIS for SNP 298 of the
      beta-2 adrenergic receptor gene.

<400> 3
gcgggcgggg cgccgtgggt cagccc                                        26

<210> 4
<211> 26
<212> DNA
<213> Artificial Sequence

<220>
<223> Description of Artificial Sequence: Allele
      specific primer b2_298_T_tMIS for SNP 298 of the

beta-2 adrenergic receptor gene.

<400> 4
gcgggcgggg cgccgtgggt cagcct 26

<210> 5
<211> 16
<212> DNA
<213> Homo sapiens

<220>
<223> Primer b2_2981 for the determination of the SNP 298
of the beta-2 adrenergic receptor gene.

<400> 5
ccgccgtggg tccgcc 16

<210> 6
<211> 17
<212> DNA
<213> Homo sapiens

<220>
<223> Primer b2_3901 for the determination of the SNP
390 of the beta-2 adrenergic receptor gene.

<400> 6
tcttgctggc acccaat 17

<210> 7
<211> 17
<212> DNA
<213> Homo sapiens

<220>
<223> Primer b2_325r for the determination of the SNP
325 of the beta-2 adrenergic receptor gene.

<400> 7
cgcgcagtct ggcaggt 17

<210> 8
<211> 18
<212> DNA
<213> Homo sapiens

<220>
<223> Primer b2_4231 for the determination of the SNP
4231 of the beta-2 adrenergic receptor gene.

<400> 8
gaccacgacg tcacgcag 18